# EUROPÄISCHE PATENTSCHRIFT

(11) **EP 1 234 498 B1**
(45) Veröffentlichungstag und Bekanntmachung des Hinweises auf die Patenterteilung: **17.09.2003**
(21) Anmeldenummer: 01104188.6
(22) Anmeldetag: 21.02.2001
(51) Int. Cl.: A01K 11/00, A61B 10/00, G01N 1/04

(54) **Vorrichtung zur Markierung von Nutztieren mit gleichzeitiger Gewebeprobenentnahme**
Device for marking farm animals with concurrent collection of tissue samples
Dispositif de marquage de bétail à collection conjointe d'un échantillon de tissu

(43) Veröffentlichungstag der Anmeldung: 28.08.2002
(73) Patentinhaber: Agrobiogen GmbH Biotechnologie, Larezhausen, 86567 Hilgertshausen (DE)
(72) Erfinder: Brem, Gottfried, 86567 Hilgertshausen (DE)
(74) Vertreter: Becker Kurig Straus

(56) Entgegenhaltungen:
- EP-A- 0 006 667
- EP-A- 1 060 662
- WO-A-99/61882
- DE-A- 19 740 429

## Beschreibung

Die vorliegende Erfindung betrifft eine Vorrichtung zur Markierung von Tieren mit gleichzeitiger Probenentnahme. Die vorliegende Erfindung betrifft insbesondere eine Vorrichtung, mit der derartige Verfahrensschritte unter Verwendung herkömmlicher Ohrmarken gleichzeitig durchgeführt werden können.

Aufgrund der sich in Europa ausbreitenden Erkrankungen von Nutztieren, wie beispielsweise die Bovine Spongiform Encephalopathie (BSE), ist von seiten der Hersteller und Verbraucher gewünscht, Informationen über die Herkunft sowie ggf. über die genetische Konstellation der Tiere geben zu können bzw. zu erhalten. Damit soll einerseits eine zuverlässige Identitäts- und Herkunftssicherung und andererseits - neben anderen tierzüchterischen Anwendungen - eine Analyse der DNA auf besondere Resistenz oder Anfälligkeit der Tiere gegen diese Erkrankungen ermöglicht werden.

Dazu müssen die Tiere einerseits, wie auch von nationalen Behörden grundsätzlich vorgeschrieben, mit einer Kennzeichnung versehen, sowie den Tieren eine Probe entnommen werden, aufgrund der die Tiere genotypisch untersucht werden können. Die Gründe einer Entnahme von Gewebeproben sind vielfältig, wie beispielsweise um
- diese Proben einzulagern und eine Gewebebank anzulegen, oder
- um aus diesen Gewebeproben DNA zu isolieren und einzulagern, oder
- um die isolierten DNAs sofort zu analysieren und die Tiere zu genotypisieren und diese Daten in einer Datenbank zu sammeln oder
- eine Kombination dieser Vorgehensweisen zu realisieren (z.B. Genotypisierung und zusätzlich Einlagern von DNA für spätere weitergehende Untersuchungen).

Gegenwärtig stellt sich auch das Problem Gewebeproben von Tieren zu gewinnen, die nicht oder nicht mehr für den menschlichen Verzehr vorgesehen sind, sondern im Rahmen beispielsweise einer Marktbereinigung vernichtet werden. Dabei ist von Bedeutung, daß von diesen Rindern Gewebeproben gewonnen werden, damit diese bzw. später die aus diesen Gewebeproben isolierte DNA für wissenschaftliche Untersuchungen im Rahmen der BSE-Forschung zur Verfügung stehen.

In der WO 98/03075 wird ein Verfahren zur Kennzeichnung und gleichzeitigen Gewinnung von Gewebeproben offenbart. Dabei wird mit der Hohlspitze einer speziell dafür konstruierten Ohrmarke beim Durchstechen des Ohres zum Anbringen der Ohrmarke eine Gewebeprobe ausgestanzt und unmittelbar in einen Probensammelbehälter eingebracht und durch das dort befindliche stark hygroskopische Molekularsieb derart ausgetrocknet, dass sie vor Isolierung der DNA jahrelang gelagert werden kann.

In der EP-A- 106 0662 wird eine Zange zum gleichzeitigen Anbringen einer Ohrmarke und Entnehmen einer Gewebeprobe offenbart.

Bei den Anwendern besteht jedoch ein Bedarf mit konventionellen Ohrmarken weiterzuarbeiten, da diese eine schon seit vielen Jahren bewährte Technik der Tierkennzeichnung darstellen und das Vertrauen von Verbänden und Tierbesitzern geniessen. So ist insbesondere "im Feld" bereits die Haltbarkeit (> 10 Jahre) der Kunststoffe unter widrigen Umweltbedingungen und die Ablesbarkeit der Beschriftung nachgewiesen und die meisten dieser Ohrmarken sind darüber hinaus "geschlossen", d.h. die Spitze des Dorns ist gänzlich von einer Kappe umgeben, so dass sie gegen Manipulationen geschützt sind. Ein weiterer Gesichtspunkt ist, daß die Ohrmarken sehr kostengünstig erhältlich sind, da sie pro Jahr weltweit mit gut etablierten und vorhandenen Produkstionssystemen in Stückzahlen von über Hundertmillionen hergestellt werden. Eine Verwendung von bereits vorhandenen Ohrmarken weist zudem den Vorteil auf, dass keine neuen Zulassungs- oder Anerkennungsverfahren für die Ohrmarken nötig sind.

Eine Aufgabe der vorliegenden Erfindung besteht daher darin, eine einfache und schnelle Markierung von Nutztieren mit gleichzeitiger Probenentnahme zu ermöglichen, wobei die gegenwärtig verwendeten Ohrmarken zum Einsatz kommen können.

Diese Aufgabe wird gelöst durch eine Vorrichtung zum gleichzeitigen Anbringen einer Ohrmarke und Entnehmen einer Gewebeprobe, welche ein erste Einrichtung zur Aufnahme einer weiblichen (Loch-) Platte und eine im wesentlichen in der Nähe der ersten Einrichtung angeordnete zweite Einrichtung zur Aufnahme eines Probenbehälters aufweist, sowie eine der ersten Einrichtung im wesentlichen gegenüberliegende dritte Einrichtung zur Aufnahme einer männlichen (Dorn-) Platte und eine der zweiten Einrichtung im wesentlichen gegenüberliegende vierte Einrichtung zur Aufnahme eines Probengewinnungsmittels, Mittel zum Zusammenführen der jeweils im wesentlichen gegenüberliegenden Einrichtungen, wobei beim Zusammenführen der ersten, zweiten und dritten bzw. vierten Einrichtung die Ohrmarke am Tier befestigt und gleichzeitig das Probengewinnungsmittel unter Mitnahme von Gewebe durch das Ohr des Tiers in den Probenbehälter geführt wird und diesen dichtend verschließt.

In den Figuren zeigt,
Fig. 1A eine Seitenansicht einer bevorzugten Ausführungsform der Erfindung, bei der eine schematisch gezeigte herkömmliche Zange zum Einziehen von Ohrmarken zwei Stifte und zwei Aufnahmeeinrichtungen zur Aufnahme einer Ohrmarke und eines Probenbehälters aufweist;
Fig. 1B eine Sicht von oben auf die Lochplatte der Ohrmarke und den daneben angeordneten Probenbehälter, wie sie in der Vorrichtung von Fig. 1A angeordnet sind;
Fig. 2A eine Ansicht von vorne auf eine weitere bevorzugte Ausführungsform der Erfindung, bei der die Aufnahmeeinrichtungen und Stifte jeweils an Backen einer Zange nebeneinander angeordnet sind;
Fig. 2B eine Ansicht der Lochplatte und des Probenbehälters von oben, wie sie in der Vorrichtung von Fig. 2A angeordnet werden;
Fig. 3 A - D einen Probenbehälter von verschiedenen Ansichten mit jeweils anderen Deckeln.

Gemäß einer bevorzugten Ausführungsform weist die erfindungsgemäße Vorrichtung die Form einer Zange auf, mehr bevorzugt, die Form einer herkömmlichen Ohrmarkenzange, wie sie gegenwärtig zum Einziehen von Ohrmarken verwendet wird. Diese Ohrmarkenzange ist nun derart modifiziert, daß sie in der Nähe der Aufnahmeeinrichtung für die Lochplatte eine weitere Aufnahmeeinrichtung für den Probenbehälter aufweist, sowie einen weiteren Stift, der der weiteren Aufnahmevorrichtung für den Probenbehälter im wesentlichen gegenüberliegt, so daß beim Zukneifen der das Probengewinnungsmittel tragende Stift dieses durch das Gewebe des Tiers hindurch unter Mitnahme einer biologischen Probe in den Probenbehälter überführt.

Das Probengewinnungsmittel ist dabei derart aufgebaut, daß es an seinem vorderen Ende so gestaltet ist, daß eine Probe aus dem Gewebe, wie beispielsweise durch Stanzen oder Schneiden oder Stechen gewonnen werden kann. Das hintere Ende des Probengewinnungsmittel ist weiter derart ausgestaltet, daß es einerseits auf die Einrichtung in der Zange paßt und weiter beim Einführen in den Probenbehälter diesen dichtend verschließt. Der Probenbehälter kann jegliche Form annehmen, insoweit er an eine entsprechende Einrichtung zur Aufnahme des Probenbehälters angepaßt ist. Vorzugsweise weist der Probenbehälter eine Lasche auf, an der eine entsprechende Markierung angebracht werden oder bereits vorgesehen sein kann.

Die Einrichtung zur Aufnahme des Probenbehälters kann in Analogie zur Einrichtung zur Aufnahme einer Lochplatte eine Vertiefung sein, in die der Probenbehälter eingeführt wird und dort ortstabil verbleibt. Dabei wird die laterale Höhe der Einrichtungen zur Aufnahme der Lochplatte und zur Aufnahme des Probenbehälters derart gewählt, daß gleichzeitig mit dem Verschluß der Ohrmarke auch der Probenbehälter mit dem Probengewinnungsmittel verschlossen wird.

Bei den erfindungsgemäßen Vorrichtungen können Gewebeprobenentnahmen grundsätzlich "vor" oder "neben" der Dornachse einer konventionellen Ohrmarke oder auch an anderen Stellen erfolgen, wobei die Bezeichnungen "vor" und "neben" jeweils in Bezug zu dem Mittel zum Zusammenführen der jeweiligen Einrichtungen zu sehen sind, d.h. dem Handteil der Zange bzw. dem Drehpunkt.

Für eine gleichzeitig mit der Ohrmarkenanbringung stattfindende Gewebeprobenentnahme "vor" der Ohrmarke muß die Zange sowohl einen zweiten, vorzugsweise metallischen, Innendorn wie auch eine zweite Aufnahme für den vorzugsweise zylindrischen/kegelstumpfförmigen Körper des Probensammelbehälters aufweisen. Beide zusätzlichen Vorrichtungen befinden sich in diesem Fall in Richtung des Drehpunktes der Zange gelegenen Positionen, also hinter den ursprünglichen, an der Spitze der Zangenschenkel positionierten Einrichtungen für die Aufnahme der konventionellen Ohrmarkenteile. Ausserdem kann an der Zange eine Rückhaltevorrichtung für die Lasche des Probensammelbehälters angebracht werden, die beispielsweise als Karabinerhaken ausgebildet ist und in der die Lochöffnung der Lasche eingelegt wird, so dass beim Entfernen der Zange vom Ohr des Tieres die über die Lochöffnung an der Zange fixierte Lasche den Sammelbehälter festhält.

Gemäß einer weiteren erfindungsgemäßen Ausführungsform kann auch eine Gewebeprobenentnahme "hinter" der Ohrmarke, also in Verlängerung der Zangenschenkel, realisiert werden, wenn die Ohrmarke beim Einziehen um mindestens 90° verdreht wird.

Eine Probenentnahme neben der Dornachse der konventionellen Ohrmarke kann beispielsweise durch eine doppelschenkelige Ausbildung des Ohrmarkenteils der Zange realisiert werden. Es ist dem Fachmann jedoch klar, daß auch eine Zange verwendet werden kann, bei der die Anordnung "nebeneinander" auf einem Schenkel realisiert wird.

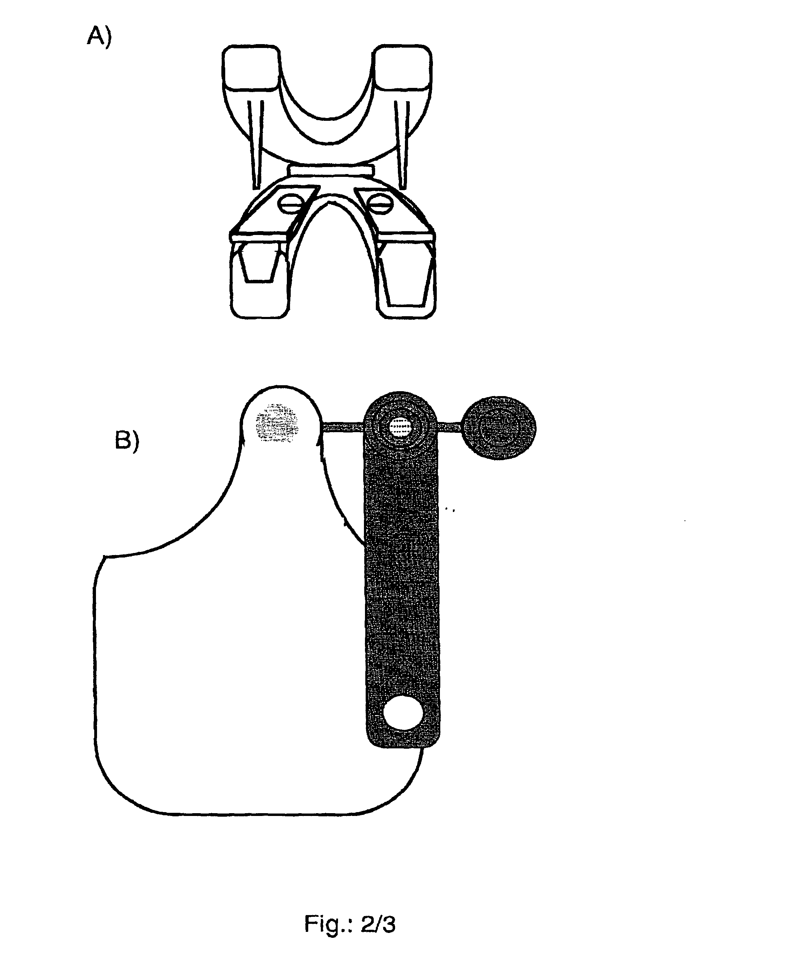

Bei der zweischenkligen Ausführungsform ist jeweils in im wesentlichen gleichem Abstand von der Mittelachse der Zange auf der einen Seite ein Schenkel der Zange konventionell ausgebildet, um die Ohrmarke zu tragen. Der Schenkel auf der anderen Seite trägt eine Aufnahmeeinrichtung für den Probensammelbehälter und eine Rückhalteeinrichtung für die Lasche des Probensammelbehälters. Für die Aufnahme des Dornteils der konventionellen Ohrmarke und der für die Gewebeausstanzung notwendigen Probenentnahmespitze (z.B. eine Hohlspitze, die beispielsweise kegelstumpfförmig ist und eine vorzugsweise eine Metallhülse mit scharfer Kante besitzt, die das Ohr durchdringt und dabei die Gewebeprobe ausstanzt ohne Teile im Ohr zu hinterlassen) hat die Zange dementsprechend ebenfalls einen Doppelschenkel (Fig 2 A).

Gegenüber einer konventionellen Probenentnahme, also einer Gewebeprobengewinnung ohne Kombination mit dem Einziehen von Ohrmarken mit den genannten Vorteilen bei der Identifikation und Konservierung wird durch die gleichzeitig mit dem Einziehen der Ohrmarke stattfindende Parallelprobennahme vor oder neben der Dornachse eine deutliche Arbeitsersparnis und eine massive Reduzierung der Fehlerquote erreicht.

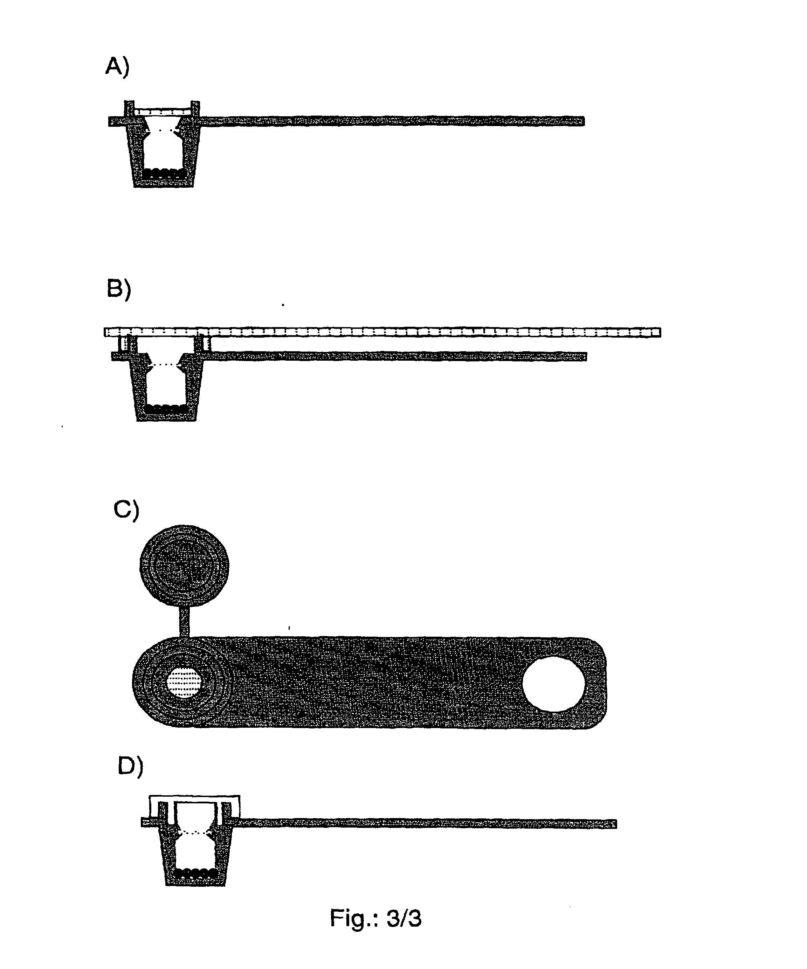

Die Probensammelbehälter können Konservierungsmittel für die biologische Probe enthalten, enthalten, wie beispielsweise Molekularsieb, das die Zerstörung/Lyse des Gewebes und der DNA verhindert. Dieses Molekularsieb kann lose in den Probensammelbehälter eingefüllt werden oder mittels thermischer Verfahren oder durch Verkleben an den Boden des Probensammelbehälter fixiert werden (Fig. 3 A, B und D).

Der Probensammelbehälter kann darüber hinaus so ausgebildet sein, dass er vor der Benutzung bereits eine geschlossene Kammer darstellt, die einen oder mehrere Deckel aufweist und bei der durch das Eindringen des Probengewinnungsmittels der Deckel durchdrungen wird, sich aber sofort wieder selbständig verschliesst (Fig. 3 A und B).

Unmittelbar nach der Probengewinnung umschliesst die Probenkammer im Normalfall das Probengewinnungsmittel und die Probe luftdicht. Als zusätzliche Sicherheit und für die spätere Bearbeitung der Gewebeproben kann bei der Herstellung des Probensammelbehälters gleichzeitig ein Deckel aus Kunststoff hergestellt werden (Fig. 3 C), der nach der Gewinnung der Probe und dem Entnehmen des Probensammelbehälters aus der Zange auf das obere Ende des Probensammelbehälters aufgesteckt/gedrückt wird und dieses luftdicht verschliesst, indem der Steg des Sammelbehälters innen und aussen von Stegen des Deckels fest umschlossen wird (Fig. 3 D). Dies stellt eine zusätzliche Sicherheit für das luftdichte Aufbewahren der Proben dar.

Zur Verbesserung der Stanzaktion kann in die obere Öffnung der Probensammelbehälter entweder ein ringförmiges Teil des den Dorn umschliessenden Teils eingelegt oder einfach eine Scheibe aus geeignetem Kunststoff (Fig. 3 A). Dadurch wird erreicht, dass die Spitze des Probengewinnungsmittels beim Zusammenführen auf ein entsprechend stabiles Gegenstück trifft und so zuverlässig ein Gewebestück ausgestanzt wird und zwar selbst dann, wenn das Ohrgewebe extrem weich und wenig Widerstand bietet, wie dies beispielsweise bei neugeborenen Lämmern oder Ferkeln der Fall ist. Ohne dieses "Widerlager" wird in vielen Fällen keine hinreichende Probe gestanzt/gewonnen, weil das Gewebe zerreisst und dem Stanzvorgang ausweicht, bevor genügend Material in die Hohlspitze des Probengewinnungsmittels aufgenommen worden ist.

Die vorliegende Erfindung betrifft auch ein Verfahren zur Markierung von und gleichzeitiger Probenentnahme bei Nutztieren, bei der eine erfindungsgemäße Vorrichtung zum Einsatz kommt.

Dabei können Teile der herkömmlichen Ohrmarken und die Probenbehälter bzw. Probengewinnungsmittel separat in die Zange geladen werden, bevor durch Zudrücken am Ohr des Tiers die Ohrmarke angebracht und gleichzeitig das Probengewinnungsmittel durch das Ohr des Tiers in den Probenbehälter überführt werden.

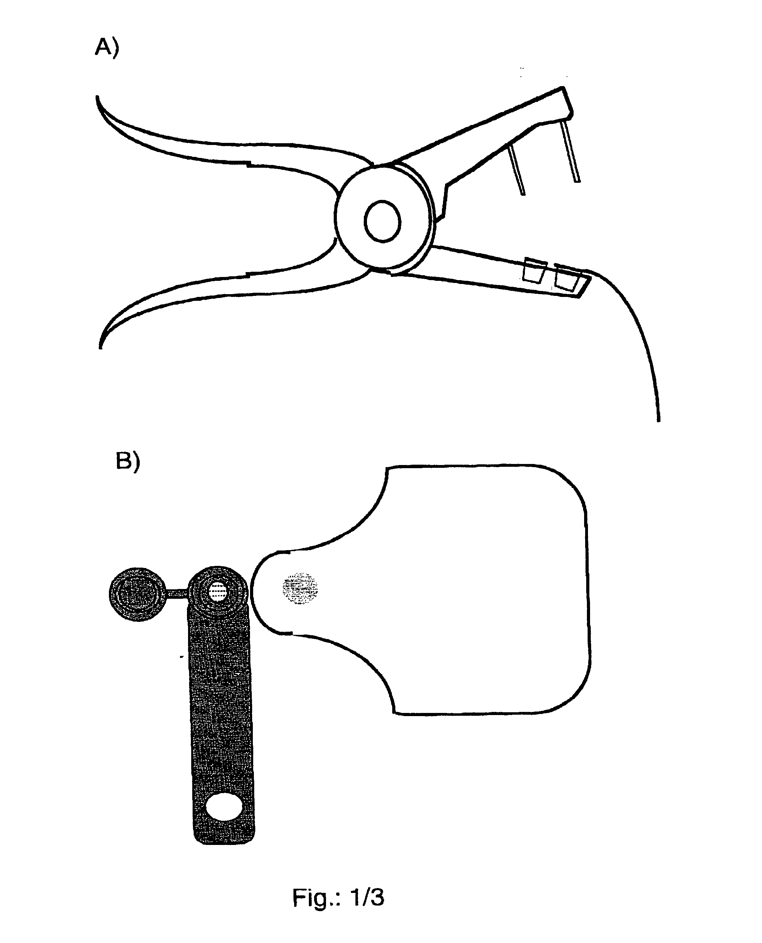

Als alternative Ausführungsform können jedoch zur Vereinfachung der Vorgehensweise Probengewinnungsmittel und Probenbehälter mit den entsprechenden Teilen der Ohrmarke verbunden sein (siehe Fig. 1 B und 2 B), so daß die Beladung der Zange vereinfacht wird. Die Fixierung/Verbindung kann erfolgen durch beispielsweise Ultraschallverschweissung, Kleben oder jede andere Form des Zusammenfügens oder der Montage, die geeignet ist, die beiden Teile derart miteinander zu verbinden, dass sie bis zum Gebrauch beeinanderbleiben und nach dem Gebrauch leicht voneinander getrennt werden können, indem der Probensammelbehälter abgerissen wird bzw. in der Zange verbleibt, wenn die Ohrmarke dem Tier eingezogen worden ist.

Ein wichtiger Punkt des hier vorgeschlagenen Verfahrens ist die Beschriftung der Probenbehälter. Wenn Probenbehälter und Ohrmarke verschiedene Nummern tragen, müssen diese Nummern mit Hilfe von Listen oder elektronisch miteinander verknüpft werden, was den Nachteil hat, dass damit ein zusätzlicher Arbeitsaufwand und Fehlerquellen bestehen. Gemäß einer bevorzugten Ausführungsform wird daher der Probensammelbehälter mit derselben Nummer versehen, die auch die Ohrmarke trägt. Dies kann auf verschiedene Weise erreicht werden. Am einfachsten werden Ohrmarke und Sammelbehälter in einem Arbeitsgang z.B. durch Laserbeschriftung markiert und die Nummern sowie Bar- und/oder Matrix-Codes gleichzeitig auf alle Teile angebracht. Wenn die Ohrmarken bereits beschriftet sind, wird die Nummer bzw. der Code von einem Lesegerät identifiziert und dann auf den Sammelbehälter kopiert.

Für die jahrelange Lagerung der Probensammelbehälter ist es vorteilhaft, diese nach dem Einsammeln in Gruppen von 100 oder 1000 Stück oder nach bestimmten Kriterien geordnet unter Vakuum in Plastikbeutel einzuschweissen. Dadurch kann sichergestellt werden, dass während der Lagerung keine negativen Einflüsse wie Wasser, Feuchtigkeit, Schmutz oder Staub auf die Probensammelbehälter einwirken können, selbst wenn in Einzelfällen die Dichtigkeit einzelner Probensammelbehälter nicht andauernd gewährleistet sein sollte. Diese Form der Probenlagerung ist sehr platz- und kostensparend: für eine Million Proben sind weniger als etwa 5 m³ Raum nötig, wobei dieser Raum keine besonderen Bedingungen hinsichtlich Lichtverhältnisse oder Trockenheit/Feuchtigkeit zu erfüllen hat.

Die Gewebeprobenentnahme bei Nutztieren parallel zum Einziehen von Ohrmarken kann auch dazu verwendet werden, um von einem Tier beim Einziehen der Ohrmarke gleichzeitig mehr als nur eine Probe (bis zu 4) zu gewinnen. Dies erlaubt es z.B. aus einer Probe sofort DNA zu isolieren und zu analysieren und die zweite Probe einzulagern und als Rückstellprobe für diverse Zwecke aufzubewahren.

In der Europäischen Gemeinschaft ist die Kennzeichnung von Rindern mit zwei Ohrmarken vorgeschrieben (siehe VO zum Schutz gegen die Verschleppung von Tierseuchen im Tierverkehr - Viehverkehrsordnung) Wenn die Gewebeprobeentnahme parallel zum Einziehen der Ohrmarken erfolgt, genügt es mitunter, wenn beim Einziehen einer der beiden Ohrmarke eine Probenentnahme vorgenommen wird. Wird in einer Population, in der alle Tiere typisiert sind, beim Ersatz von ausgefallenen oder verlorenen Ohrmarken in allen Fällen parallel mit dem Einziehen der Ersatzohrmarke eine Gewebeprobe gewonnen, dann kann durch Genotypisierung festgestellt werden, ob die Ersatzohrmarke tatsächlich dem Tier eingezogen worden ist, bei dem die Ohrmarke als verloren gemeldet worden ist.

Zur Gewinnung von mehr als nur einer Probe kann nun die erfindungsgemäße Vorrichtung dazu verwendet werden an beiden Ohren des Nutztiers jeweils eine Probe zu gewinnen. Dies erlaubt es z.B. aus einer Probe sofort DNA zu isolieren und zu analysieren und die zweite Probe einzulagern und als Rückstellprobe für diverse Zwecke aufzubewahren. Sollen mehr als nur zwei proben gewonnen werden, so können die im Handel erhältlichen Typi-Fix®-Ohrmarken (siehe WO 98/03075, die hiermit zur Beschreibung der Konstruktionsmerkmale der Typifix-Ohrmarken unter Bezugnahme mit aufgenommen wird) eingesetzt werden, mit denen eine gleichzeitige Probenentnahme und Markierung des Tiers an einer Stelle (an der Stelle der Ohrmarke) ermöglicht wird. In diesem Fall werden also bei der Markierung des Tieres an einem Ohr zwei Proben gewonnen, eine mit Hilfe der Typi-Fix®-Ohrmarke und eine mit Hilfe des separaten Probensammelbehälters und Probengewinnungsmittels vor oder neben der Typi-Fix®-Ohrmarke.

Ein Vorteil dabei ist auch, daß, dass diejenige Probe, die mittels Typi-Fix®-Ohrmarke gewonnen wurde, nicht sofort eingesammelt werden muss, sondern am Tier verbleiben kann. In diesen Fällen kann es vorteilhaft sein, die Lasche der Typi-Fix®-Ohrmarke etwas zu kürzen und auf das Loch am Ende der Lasche zu verzichten. Dann kann der mit dem weiblichen Teil der Ohrmarke verbundene Probensammelbehälter mit der eingepackten Probe an der Ohrmarke und damit am Tier verbleiben. Zu einem späteren Zeitpunkt, wenn eine weitere DNA-Identifizierung des Tieres gewünscht wird, kann der Probensammelbehälter, der natürlich ebenfalls mit der Identität des Tieres bzw. der Ohrmarkennummer des Tieres versehen ist, eingesammelt und analysiert werden.

Das könnte vor allem dann gewünscht sein, wenn das Tier z.B. aus der EU exportiert wird. Eine andere Möglichkeit ist, dass diese identifizierte Probe beim Schlachten des Tieres routinemässig eingesammelt und analysiert wird. Eine andere Nutzung kann darin bestehen, dass diese konservierte Probe von der Ohrmarke entfernt und am Schlachtkörper fixiert wird und diesen bis zur Zerlegung, während einer eventuellen Tiefgefrierlagerung oder auf Langzeittransporten von Südamerika nach Europa begleitet oder beim BSE-Test als Kontrollgewebe für die Identitätsund Herkunftssicherung der Himprobe Verwendung findet.

Anstelle eines einfachen Probensammelbehälter kann auch eine sogenannte Mini-Flagge eingesetzt werden, um zu einem späteren Zeitpunkt feststellen zu können, daß von dem Tier eine Probe Gewonnen wurde. Dabei wir eine Typi-Fix-Ohrmarke in verkleinertem Maßstab neben einer herkömmlichen Ohrmarke eingesetzt, so daß neben der Ohrmarke noch die Mini-Flagge verbleibt und anzeigt, daß von diesem Tier bereits eine biologische Probe gewonnen wurde.

Die Erfindung wird nun anhand der folgenden Beispiele weiter erläutert.

### Beispiel 1:

Eine Zange der Firma Merko aus Belgien wurde durch Anbringung einer zweiten Aufnahme und eines zweiten Metalldornes umgebaut. Ohrmarken der Firma Allflex wurden mit Probensammelbehältern des Typi-Fix®-Systems so verklebt, dass die Probensammelbehälter vor die Ohrmarken zu liegen kamen. Die auf den konventionellen Ohrmarken vorhandenen Nummern wurden abgelesen und auf die Probensammelbehälter aufgebracht.

Bei fünfzig Probenentnahmen aus Rinderohren (umgebaute Zange der Fa. Merko, nachbeschriftete Ohrmarke der Fa. Allflex, Typi-Fix®-Sammelbehälter mit eingelegtem Stanzdeckel und eingeschweisstem Molekularsieb) konnte in allen Fällen gleichzeitig mit dem Einziehen der Ohrmarke eine Gewebprobe gewonnen werden. Nach 2 monatiger Lagerung wurde aus den Gewebeproben in den Typi-Fix®-Probensammelbehältern mit Hilfe des Isolationskits von Machery & Nagel DNA isoliert. Im Durchschnitt wurden 30µg DNA isoliert. In allen Fällen konnten bei der anschliessenden Mikrosatellitenanalyse die Tiere eindeutig genotypisiert werden.

Ohrmarken der Fa. Caisley wurden mit Probensammelbehältern durch Ultraschallschweissen in der Weise verbunden, dass die Probensammelbehälter neben die Dornachse der konventionellen Ohrmarke zu liegen kamen. Ohrmarken und Sammelbehälter wurden gleichzeitig mit jeweils identen Nummern beschriftet. Durch Verbindung der Frontteile der Schenkel zweier Zangen der Fa. Hauptner wurde eine Zange hergestellt, mit der neben der Ohrmarke eine Probenentnahme durchgeführt werden konnte.

Aus allen zwanzig von Lämmern in der ersten Lebenswoche gezogenen Proben (umgebaute Zange der Fa. Hauptner, neu beschriftete Ohrmarke der Fa. Caisley, Typi-Fix®-Sammelbehälter mit eingelegtem Stanzdeckel und eingeschweisstem Molekularsieb) wurde DNA (im Durchschnitt 20µg DNA) isoliert und genotypisiert.

## Patentansprüche

1. Vorrichtung zum gleichzeitigen Anbringen einer Ohrmarke und Entnehmen einer Gewebeprobe, welche
ein erste Einrichtung zur Aufnahme einer Lochplatte und eine im wesentlichen in der Nähe der ersten Einrichtung angeordnete zweite Einrichtung zur Aufnahme eines Probenbehälters aufweist, sowie
eine der ersten Einrichtung im wesentlichen gegenüberliegende dritte Einrichtung zur Aufnahme einer Dornplatte und eine der zweiten Einrichtung im wesentlichen gegenüberliegende vierte Einrichtung zur Aufnahme eines Probengewinnungsmittels,
Mittel zum Zusammenführen der jeweils im wesentlichen gegenüberliegenden Einrichtungen,
wobei beim Zusammenführen der ersten, zweiten und dritten bzw. vierten Einrichtung die Ohrmarke am Tier befestigt und gleichzeitig das Probengewinnungsmittel unter Mitnahme von Gewebe durch das Ohr des Tiers in den Probenbehälter geführt wird und diesen dichtend verschließt.

2. Vorrichtung nach Anspruch 1, worin die erste und zweite Einrichtung zur Aufnahme der Lochplatte bzw. des Probenbehälters angepaßte Vertiefungen sind.

3. Vorrichtung nach Anspruch 1 oder 2, worin die dritte und vierte Einrichtung zur Aufnahme der Dornplatte bzw. des Probengewinnungsmittels angepaßte Stifte sind.

4. Vorrichtung nach einem der vorhergehenden Ansprüche, worin die Einrichtungen zur Aufnahme der Ohrmarkenteile und die Einrichtungen zur Aufnahme des Probenbehälters und des Probengewinnungsmittels in Bezug zu dem Mittel zum Zusammenführen der Einrichtungen hintereinander oder nebeneinander angeordnet sind.

5. Verwendung einer Einrichtung nach einem der vorhergehenden Ansprüche zur Markierung von Nutztieren mit gleichzeitiger Probenentnahme.

6. Verwendung nach Anspruch 5, wobei die Nutztiere Rinder, Schweine, Ziegen, Schafe oder Kaninchen sind.

7. Verfahren zur Markierung von und gleichzeitigen Entnahme einer biologischen Probe aus einem Nutztier, bei dem eine Vorrichtung nach einem der vorhergehenden Ansprüche eingesetzt wird.

8. Verfahren zur Markierung nach Anspruch 7, wobei mehr als eine Probe von dem Tier gewonnen wird.

9. Verfahren nach Anspruch 8, wobei mindestens ein Probenbehälter von dem Tier genommen und mindestens einer der verbleibenden Probenbehälter am Tier verbleibt.

10. Verfahren nach einem der Ansprüche 7 bis 9, wobei der Probenbehälter in Form einer Typi-Fix-Ohrmarke vorliegt.

## Claims

1. A device for simultaneously fixing an ear tag and collecting a tissue sample, which has
a first equipment for receiving an aperture plate and a second equipment arranged essentially in the proximity of the first equipment for receiving a sample container, as well as
a third equipment for receiving a spike plate essentially opposite to the first equipment and a fourth equipment essentially opposite to the second equipment for receiving a sample collecting means,
means for bringing together the equipments being essentially opposite to each other, wherein upon bringing together the first, second, and third and fourth equipment, respectively, the ear tag is fixed on the animal and simultaneously the sample collecting means is guided through the ear of the animal into the sample container therein taking away tissue and tightly sealing the sample container up.

2. The device according to claim 1, wherein the first and the second equipment are recesses adapted for receiving the hole punch and the sample container, respectively.

3. The device according to claim 1 or 2, wherein the third and fourth equipment are pins adapted for receiving the spike plate and sample collecting means, respectively.

4. The device according to any of the preceding claims, wherein the equipments for receiving the ear tag parts and the equipments for receiving the sample container and the sample collecting means are arranged one after the other or side by side with respect to the means for bringing together the equipments.

5. Use of an equipment according to any of the preceding claims for simultaneously labeling livestock and taking a sample.

6. The use according to claim 5, wherein livestock is cattle, pigs, goats, sheep, or rabbits.

7. A process for labeling livestock and simultaneously taking a biological sample therefrom, wherein an equipment according to any of the preceding claims is used.

8. The process for labeling according to claim 7, wherein more than one sample is collected from the animal.

9. The process according to claim 8, wherein at least one sample container is taken from said animal and wherein at least one of the remaining sample containers remain with the animal.

10. The process according to any of claims 7 to 9, wherein the sample container is in the form of a Typi-Fix-ear tag.

## Revendications

1. Dispositif pour l'apposition d'une marque auriculaire simultanément au prélèvement d'un échantillon de tissus, comprenant
un premier élément conformé pour coopérer avec une plaque percée et un deuxième élément, situé sensiblement à proximité dudit premier élément et conformé pour coopérer avec un récipient pour échantillon, ainsi qu'
un troisième élément faisant sensiblement face audit premier élément et conformé pour coopérer avec un embout et un quatrième élément faisant sensiblement face audit deuxième élément et conformé pour coopérer avec un moyen de prélèvement d'échantillon, ainsi que des moyens d'assemblage de chacun des éléments faisant sensiblement face l'un à l'autre,
dans lequel la réunion du premier, deuxième et troisième respectivement quatrième élément entraîne la fixation de la marque auriculaire à l'animal et simultanément le déplacement du moyen de prélèvement d'échantillon au travers de l'oreille de l'animal avec prise de tissus au passage, à l'intérieur du récipient pour échantillon et fermeture de ce dernier de manière hermétique.

2. Dispositif selon la revendication 1, dans lequel les premier et deuxième éléments sont des cavités adaptées à être conformées pour coopérer avec la plaque percée, respectivement le récipient pour échantillon .

3. Dispositif selon la revendication 1 ou 2, dans lequel les troisième et quatrième éléments sont des broches adaptées à être conformées pour coopérer avec l'embout, respectivement le moyen de prélèvement d'échantillon.

4. Dispositif selon l'une des revendications précédentes, dans lequel les éléments conformés pour coopérer avec les pièces de la marque auriculaire et les éléments conformés pour coopérer avec le récipient pour échantillon et le moyen de prélèvement dudit échantillon se situent, par rapport au moyen d'assemblage desdits éléments, les uns derrière les autres ou les uns à côté des autres.

5. Utilisation d'un dispositif selon l'une des revendications précédentes pour le marquage du bétail et le prélèvement simultané d'échantillon.

6. Utilisation selon la revendication 5, le bétail comprend les bovidés, les porcs, les chèvres, les moutons et les lapins.

7. Procédé de marquage et de prélèvement simultané d'échantillon biologique d'un bétail par la mise en oeuvre d'un dispositif selon l'une des revendications précédentes.

8. Procédé de marquage selon la revendication 7, dans lequel on recueille plus d'un échantillon par animal.

9. Procédé selon la revendication 8, dans lequel au moins un récipient pour échantillon est retiré de l'animal et au moins un récipient pour échantillon reste fixé à l'animal.

10. Procédé selon l'une des revendications 7 à 9, dans lequel ledit récipient pour échantillon se présente sous forme d'une marque auriculaire « Typi-Fix ».
